# EUROPEAN PATENT APPLICATION

(11) **EP 4 628 123 A1**
(43) Date of publication of application: **08.10.2025**
(21) Application number: 25167848.8
(22) Date of filing: 01.04.2025
(51) Int. Cl.: A61M 5/172

(54) **SYSTEMS AND METHODS FOR OPTICALLY SENSING ANALYTE CONCENTRATIONS WITHIN MEDICAMENT DELIVERY DEVICES**

(30) Priority: 05.04.2024 US 202463575206 P; 07.03.2025 US 202519073834
(71) Applicant: Medtronic MiniMed, Inc., Northridge, CA 91325-1219 (US)
(72) Inventor: Tong, Davy T., Northridge, 91325 (US); Fusselman, Hsi C., Northridge, 91325 (US); Zhang, Guangping, Northridge, 91325 (US); Romo-Anselmo, Evan M., Northridge, 91325 (US); Chattaraj, Sarnath, Northridge, 91325 (US); Lorenz Marckmann, Jennifer L., Northridge, 91325 (US)
(74) Representative: Zimmermann & Partner Patentanwälte mbB

(57) **Abstract**

Systems and methods for optically sensing medicament concentrations within medicament delivery devices are disclosed. A medicament delivery device can include a fluid path comprising a reservoir in fluid communication with an outlet port, in which the device is actuatable to drive fluid through the fluid path, from the reservoir and out of the outlet port. A sensor assembly of the delivery device is configured to determine concentration of an analyte in the fluid. The sensor assembly includes an optical emitter configured to emit photons towards a fluid sample within the fluid path, an optical detector configured to receive photons passed through and/or reflected from the fluid sample and provide a detector signal, and one or more signal processing components configured to receive the detector signal and, based at least in part on the detector signal, provide an indication of the analyte concentration in the fluid sample.

## Description

### CROSS-REFERENCE TO RELATED APPLICATIONS

This application claims the benefit of priority to U.S. Provisional Application No. 63/575,206, filed April 5, 2024 and 19/073,834 filed March 7, 2025 which is incorporated herein by reference in its entirety.

### TECHNICAL FIELD

The present technology relates generally to medical devices, and more particularly, to systems and methods for sensing analyte concentrations and/or compositions in medicament delivery devices.

### BACKGROUND

Insulin delivery devices, such as insulin pumps, have become increasingly popular for managing diabetes by providing a convenient and accurate means of administering insulin to patients. These devices allow users to adjust insulin delivery based on their individual needs and requirements. If a certain insulin delivery device is designed to accommodate different concentrations of insulin, this presents a potential risk to users. For example, a given tethered and/or patch pump may be labeled to be compatible with insulin concentrations of 100 units per milliliter (U100) as well as concentrations of 500 units per milliliter (U500) and can be up to 1000 units per milliliter (U1000).

One significant challenge associated with insulin delivery devices that can accommodate various insulin concentrations is the possibility of users failing to properly adjust the pump settings for a given insulin concentration. This oversight can lead to either an over-dosing or under-dosing of insulin dispensed by the pump, which may result in serious health consequences for the user. Incorrectly adjusted pump settings can cause hyperglycemia or hypoglycemia, both of which can have detrimental effects on a patient's well-being.

Therefore, there is a need for a system that can accurately determine the concentration of insulin within an insulin delivery device and ensure that the device's settings are properly adjusted to match the detected insulin concentration. Such a system can help mitigate the risks associated with user error and improve the overall safety and efficacy of insulin delivery devices.

### SUMMARY

Generally, in some embodiments in accordance with the present technology, a medicament delivery device includes a fluid path and a sensor assembly. The fluid path comprises reservoir in fluid communication with an outlet port, and the device is actuatable to drive fluid through the fluid path, from the reservoir and out of the outlet port. The sensor assembly is configured to determine concentration of an analyte in the fluid. The sensor assembly includes an optical emitter configured to emit photons towards a fluid sample within the fluid path, an optical detector configured to receive photons passed through and/or reflected from the fluid sample and provide a detector signal, and one or more signal processing components configured to receive the detector signal, and, based at least in part on the detector signal, provide an indication of the analyte concentration in the fluid sample.

In some aspects, the analyte comprises insulin. The optical emitter can be configured to emit photons within a wavelength range between about 250-350 nm. Among examples, the detector is configured to detect photons passing through the fluid sample, and the signal processing components are configured to provide the indication of analyte concentration based on absorption of the photons by the fluid sample. In some implementations, a greater absorption of the photons by the fluid sample indicates a lower concentration of the analyte in the fluid sample.

In some aspects, the detector is configured to detect scattered photons reflected from medicament within the fluid sample over time to produce a time-domain signal, and the signal processing components are configured to provide the indication of analyte concentration based on the time-domain signal. The signal processing components can include a digital autocorrelator that correlates intensity fluctuations in detected photons over time.

Optionally, the device further comprises a reference sample that is separate from the fluid path for calibrating the sensor assembly, in which the reference sample contains fluid with no analyte therein. The reference sample can be housed within a receptacle made of the same material as a portion of the fluid path containing the fluid sample.

In some aspects, the optical emitter is configured to emit photons within a predefined wavelength range, and a portion of the fluid path containing the fluid sample comprises a material substantially translucent to photons within the predefined wavelength range. The material can include quartz, topaz, or a combination thereof.

In some aspects, the signal processing components are further configured to, based at least in part on the indication of the analyte concentration in the fluid sample, perform further actions. The further actions can include: causing an alarm to be output to a user, causing a dispensation mechanism to be adjusted, wherein the adjustment varies an amount or rate of fluid dispensed via the medicament delivery device, or inhibiting dispensation of fluid via the medicament delivery device. In various examples, the medicament delivery device can include a wearable pump or a pen injector.

Generally, in some embodiments in accordance with the present technology, a method comprises disposing fluid comprising medicament within a fluid path of a medicament delivery device, directing photons towards a fluid sample within the fluid path, detecting photons transmitted through and/or scattered from the fluid sample, and, based on the detected photons, providing an indication of an analyte concentration in the fluid sample.

In some embodiments, the analyte comprises insulin. Directing photons towards the sample can include directing photons within a wavelength range of between about 250-350 nm. In some examples, providing the indication of analyte concentration in the fluid sample based on the detected photons comprises determining the analyte concentration based on absorption of the photons by the fluid sample. Optionally, providing the indication of analyte concentration in the fluid sample based on the detected photons comprises determining the analyte concentration based on a time-domain signal of the detected photons.

In some embodiments, the method further includes, based at least in part on the indication of the analyte concentration in the fluid sample, causing an alarm to be output to a user, causing a dispensation mechanism to be adjusted, wherein the adjustment varies an amount or rate of fluid dispensed via the medicament delivery device, and/or inhibiting dispensation of fluid via the medicament delivery device.

Generally, in some embodiments in accordance with the present technology, a device includes a means for containing a fluid comprising a medicament, a means for dispensing the fluid from the fluid-containing means, and means for sensing an analyte concentration of the fluid. The sensing means includes means for emitting photons towards a fluid sample within the device, means for detecting photons from the fluid sample within the device, and means for processing a signal from the detecting means and providing an indication of the analyte concentration.

In some embodiments, the means for emitting photons comprises a light source. The light source can be a coherent light source, such as a laser. In some embodiments, the means for detecting photons comprises an optical detector. Optionally, the means for processing signals comprises signal processing circuitry.

Other technical features may be readily apparent to one skilled in the art from the following figures, descriptions, and claims.

### BRIEF DESCRIPTION OF THE DRAWINGS

Many aspects of the present disclosure can be better understood with reference to the following drawings. The components in the drawings are not necessarily to scale. Instead, emphasis is placed on illustrating clearly the principles of the present disclosure.
FIG 1 is a schematic block diagram of a fluid delivery device including an optical sensor assembly in accordance with several embodiments of the present technology.
FIG. 2 is a perspective view of an optical sensor assembly in accordance with several embodiments of the present technology.
FIG. 3 is a perspective view of an optical sensor assembly and a calibration assembly in accordance with several embodiments of the present technology.
FIG. 4A is a graph of optical transmittance over a range of wavelengths for different samples having varying concentrations of insulin.
FIG. 4B is a graph of percent differences in optical transmittance over a range of wavelengths for different fluid samples having varying concentrations of insulin.
FIG. 5 is a schematic perspective view of an optical sensor assembly in accordance with several embodiments of the present technology.
FIG. 6 is a schematic perspective view of an optical sensor assembly in accordance with several embodiments of the present technology.
FIG. 7 is a perspective view of a tethered pump subassembly, specifically a reservoir and infusion set, for medicament delivery in accordance with embodiments of the present technology.
FIG. 8 is a perspective view of interior components of a patch pump for medicament delivery in accordance with embodiments of the present technology.
FIG. 9A is a perspective view of a pen injector for medicament delivery in accordance with embodiments of the present technology.
FIG. 9B is an exploded perspective view of the pen injector shown in FIG. 8A.

### DETAILED DESCRIPTION

### I. Overview

The present technology relates to systems and methods for optically determining the concentration of a medicament within a fluid delivery device, such as for detecting insulin concentration within an insulin pump or other suitable insulin delivery device. In some implementations, a fluid delivery device utilizes light to detect insulin concentration, providing a convenient and accurate means of monitoring and/or adjusting insulin delivery.

In one aspect, a fluid delivery device includes various fluid delivery components, such as a reservoir, an outlet port, and a fluid conduit extending therebetween, with at least one fluid sample region for analysis. In some examples, the fluid can include an analyte, such as insulin or other medicament, as well as one or more diluents. An optical emitter is configured to direct photons to the fluid sample region, while an optical detector is configured to receive photons passing through and/or reflected from the fluid sample region. Signal processing component(s) are configured to analyze the signal from the optical detector and obtain an indication of the concentration of the analyte, such as insulin or other medicament, within the fluid sample region.

The present technology offers several advantages, for instance providing the ability to accurately determine insulin concentration in a non-contact, non-invasive manner, enabling further miniaturization of insulin delivery devices and longer wear times for patients by facilitating the use of higher-concentration insulin. By ensuring proper adjustment of device settings based on detected insulin concentration, the technology improves the safety and efficacy of insulin delivery, reducing the risk of over- or under-dosing due to user error.

### II. Example Optical Sensor Assemblies for Medicament Delivery Devices

### A. Optical Sensor Assembly Overview

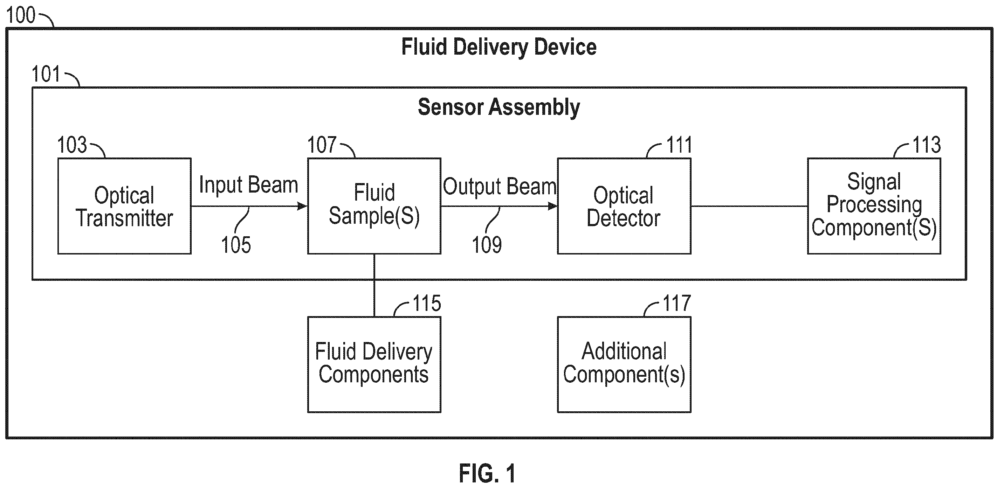

Figure 1 illustrates a schematic block diagram of a fluid delivery device 100 according to some embodiment of the present technology. The fluid delivery device 100 comprises a sensor assembly 101 and fluid delivery components 115. The fluid delivery components 115 can include, for instance, a reservoir in fluid communication with an outlet port (e.g., a port coupled to an external fluid conduit, a cannula configured to penetrate a user's skin, or any other suitable outlet port). The fluid delivery device 100 is actuatable to drive fluid through the fluid path, for instance driving fluid from the reservoir and out of the device 100 via the outlet port. The fluid delivery device 100 also includes a fluid sample 107, which can be a portion of the fluid path containing the fluid to be analyzed by the sensor assembly 101. For instance, the fluid sample 107 can be a portion of the fluid contained within a sample portion of a reservoir, an internal conduit, the outlet port, or any other suitable location within the fluid delivery device 100.

The sensor assembly 101 is configured to determine the concentration of an analyte within the fluid sample(s) 107. In various examples, the sensor assembly 101 is configured to determine the concentration of an analyte (e.g., insulin, other medicament of interest, or any suitable component of the fluid) within the fluid sample 107. The sensor assembly 101 comprises an optical emitter 103, which is configured to emit photons as an input beam 105 towards the fluid sample 107 within the fluid path.

After the input beam 105 interacts with the fluid sample 107, an output beam 109 is generated, which comprises photons that have passed through and/or reflected from the fluid sample 107. The sensor assembly 101 further comprises an optical detector 111 configured to receive the photons of the output beam 109 and provide a detector signal to one or more signal processing components 113. These signal processing components 113 can be configured to receive the detector signal from the optical detector 111 and, based at least in part on the detector signal, provide an indication of the analyte concentration in the fluid sample 107.

In some embodiments, the optical detector 111 is configured to detect photons passing through the fluid sample 107, and the signal processing components 113 are configured to determine the analyte (e.g., medicament) concentration based on absorption, or conversely the transmittance, of the photons by the fluid sample 107. As described in more detail below, within certain wavelength ranges (e.g., between about 250-350 nm, or about 290-300 nm), an increased transmittance of photons indicates a higher concentration of insulin within the fluid sample 107.

In some embodiments, the optical emitter 103 is configured to direct photons onto the fluid sample 107, and the optical detector 111 is configured to detect scattered photons reflected from the analyte (e.g., medicament) within the fluid sample 107 over time. As described in more detail below, for this approach the signal processing components 113 can be configured to determine the analyte concentration based on the time-domain signal (e.g., by correlating intensity fluctuations in detected photons over time).

The fluid delivery device 100 may further comprise additional component(s) 117, which may include a reference sample that is separate from the fluid path for calibrating the sensor assembly 101, wherein the reference sample contains fluid with no analyte therein. The reference sample may be housed within a receptacle made of the same material as the portion of the fluid path containing the fluid sample 107. The additional components 117 can also include communication components (e.g., wireless transceivers), drive mechanisms for adjusting dispensation of fluid via the fluid delivery components 115, and any other suitable components of a fluid delivery device 100.

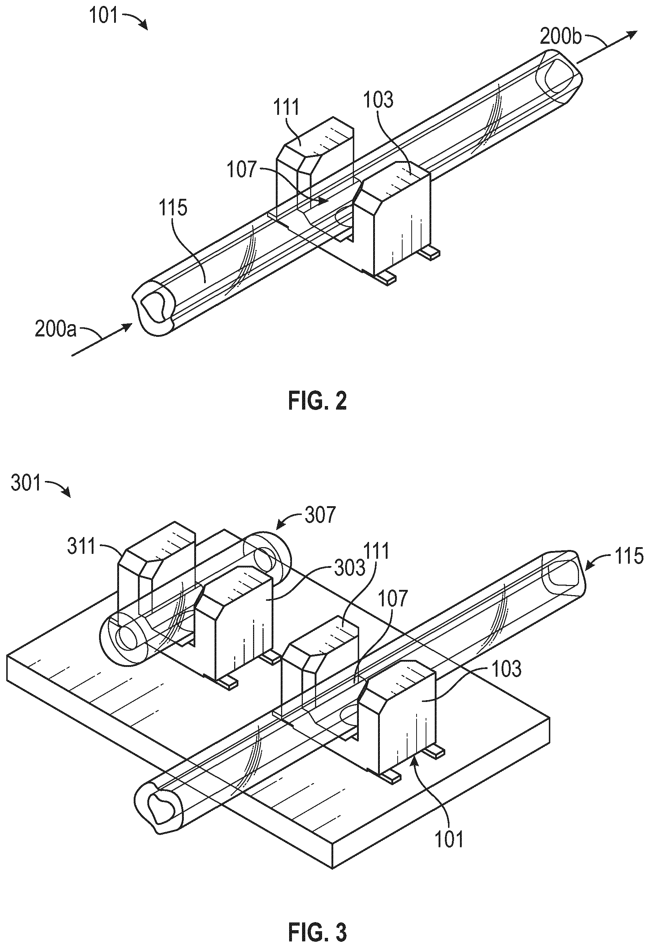

Figure 2 illustrates a perspective view of an example implementation of the optical sensor assembly 101 described above with respect to Figure 1. As depicted in Figure 2, the optical sensor assembly 101 includes a fluid sample 107 which is a portion of a tubular conduit 115 through which fluid can flow along a direction indicated by arrows 200a and 200b. On opposing sides of the fluid sample 107 are arranged an optical emitter 103 and an optical detector 111. In operation, photons emitted via the optical emitter 103 pass through the fluid sample 107 and are received via the optical detector 111. The optical detector 111 can generate a detector output signal based on the received photons, and this signal can be processed (e.g., via signal processing components 113) to render concentration determinations. Although Figure 2 illustrates an example configuration of the optical sensor assembly 101 in which the fluid sample 107 is a portion of a tubular conduit 115, in various examples the fluid sample 107 can be disposed in any suitable position within the fluid delivery device 100. For instance, the fluid sample 107 may be within the reservoir, along the outlet port, or along any suitable portions of the fluid path.

### B. Example Fluid Path Materials for Fluid Sample Region

The fluid sample 107, which can be part of the fluid path of the fluid delivery device 100, should be retained within a material that is substantially transparent to the specific wavelength(s) of light used by the sensor assembly 101. In the case of UV absorption spectroscopy using a wavelength of 295 nm, as an example, the material is relatively more transparent to this wavelength to enable the input beam 105 to pass through the fluid sample 107 and reach the optical detector 111 as the output beam 109 than at other wavelengths. Several materials that exhibit good transparency at wavelengths below 325 nm include quartz (SiO₂), sapphire (Al₂O₃), calcium fluoride (CaF₂), UV-grade fused silica (a high-purity, amorphous form of silicon dioxide), magnesium fluoride (MgF₂), cyclic olefin copolymer (COC) resins, poly methyl methacrylate (PMMA) resins such as acrylic, or any other suitable material, both inorganic and organic. In various examples, the material containing the fluid sample 107 can transmit, within a wavelength range of interest, the increase in the transmittance between analytes of varying insulin concentration is well above the noise floor and is typically about one order of magnitude greater than noise for every increase in concentration of 100 units of insulin per mL.

In some implementations, only a small portion of the overall fluid path that includes the fluid sample 107 comprises the transparent material. Additionally or alternatively, all or substantially all of the fluid path may be formed by the transparent material. The choice of material will depend on factors such as the specific design requirements and wavelengths of interest, cost, and availability.

In some examples, additional material can be provided within the fluid path to facilitate optical analysis of the fluid sample. For instance, silica particles (or other suitable material) can be disposed within the fluid path at or adjacent to the site of the fluid sample 107. Due to the surface properties of silica, specifically a polar surface due to the presence of silanol groups (Si-OH), various components of the fluid sample may interact with the silica through different mechanisms, such as hydrogen bonding, dipole-dipole interactions, and electrostatic interactions. These interactions can lead to differential retention of the analytes, such that compounds with stronger interactions with the silica surface are retained for longer, while those with weaker interactions pass earlier. This differential retention can be used to separate components of the fluid sample for analysis.

### C. Example Optical Emitters

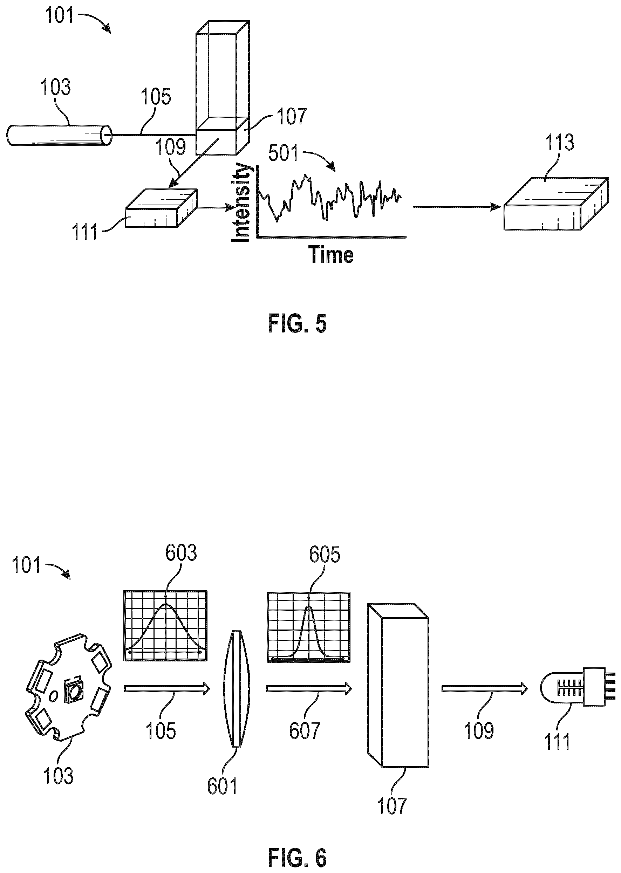

As noted above, the optical emitter 103 is responsible for generating the light that interacts with the analyte in the fluid sample, and its characteristics can significantly impact the performance and reliability of the sensor assembly 101. While various light sources are described herein, it will be appreciated that the optical emitter 103 can also include auxiliary optical components, such as lenses, mirrors, gratings, filters, etc. so as to achieve a desired output beam of photons, in terms of intensity, wavelength, and direction. The usage of a filter in combination with the optical emitter 103 is described in more detail below with respect to Figure 6. In various examples, the optical emitter 103 can be configured to provide an input beam 105 of photons within a wavelength range of 250-350 nm, or between about 275-325 nm, or between about 290-300 nm, or about 295 nm. In various examples, the optical emitter 103 is configured to provide an input beam 105 of photons having a wavelength of less than about 350 nm, 340 nm, 330 nm, 320 nm, 310 nm, 300 nm, 290 nm, 280 nm, 270 nm, 260 nm, or less.

Lasers may be used as optical emitters 103 for the optical sensor assembly 101 due to their unique properties and advantages. Lasers emit highly coherent, monochromatic, and collimated light, which makes them suitable for precise and selective illumination of the fluid sample. The narrow spectral bandwidth of laser light enables sensitivity and specificity of the concentration measurement.

Several types of lasers can be employed in the optical sensor assembly 101, depending on the specific requirements of the application. For example, diode lasers, which are compact, efficient, and cost-effective, are well-suited for integration into portable medicament delivery devices. Diode lasers are available in a wide range of wavelengths, from the ultraviolet (UV) to the near-infrared (NIR) region, allowing for the selection of the optimal wavelength for the specific medicament and optical sensing technique employed.

In addition to lasers, other types of optical emitters can also be employed in the optical sensor assembly 101, depending on the specific requirements and constraints of the application. For example, light-emitting diodes (LEDs) are another compact and cost-effective option. LEDs may be used in conjunction with bandpass filters to achieve a desired wavelength range for the input beam 105.

### D. Example Optical Detectors

As noted previously, the optical detector 111 is responsible for converting the light that has interacted with the fluid sample 107 (e.g., the output beam 109) into an electrical signal that can be processed and analyzed to determine the analyte concentration. Different types of optical detectors may be more suitable for different sensing modalities, such as absorption spectroscopy or dynamic light scattering, depending on their specific characteristics and performance.

For absorption spectroscopy, which is based on measuring the attenuation of light as it passes through the fluid sample, the optical detector 111 can take the form of a photointerrupter or optical switch. The optical detector 111 can include, for instance, a photodiode, which is a semiconductor device that generates an electrical current proportional to the intensity of the incident light. They are compact, cost-effective, and available in a wide range of wavelength sensitivities, making them well-suited for integration into the optical sensor assembly 101. Silicon photodiodes can be particularly suitable for applications in the UV wavelength range.

For dynamic light scattering, which relies on measuring the fluctuations in the intensity of scattered light over time, the optical detector 111 can include one or more photomultiplier tubes (PMTs) or avalanche photodiodes (APDs). PMTs are highly sensitive detectors that can amplify weak light signals by converting incident photons into electrons and multiplying them through a series of dynodes. This high sensitivity makes PMTs ideal for detecting the small changes in scattered light intensity associated with the Brownian motion of the analyte particles in the fluid sample. APDs operate by converting incident photons into electron-hole pairs and amplifying the resulting electrical signal through a process called avalanche multiplication. This enables APDs to achieve high gain and fast response times, making them well-suited for detecting the rapid fluctuations in scattered light intensity. Like PMTs, APDs are available in a range of wavelength sensitivities and can be selected based on the specific requirements of the application.

The orientation between the optical emitter 103 and the optical detector 111 in the optical sensor assembly 101 is another factor that can significantly impact the performance and reliability of the optical sensor assembly 101. The desired orientation depends on the specific sensing modality employed, as different techniques rely on measuring different aspects of the light-sample interaction. For instance, for absorption spectroscopy, a straight-line, 180-degree transmission geometry can be most appropriate, while for dynamic light scattering, a 90-degree side-scattering geometry may be preferred. By optimizing the orientation of the emitter and detector for the specific sensing modality, it is possible to maximize the sensitivity, reliability, and accuracy of the concentration monitoring system in the various medicament delivery devices described above. In various examples, the optical detector 111 can be arranged directly opposite the fluid sample 107 and in line with the input beam 105. Alternatively, the optical detector 111 can be arranged at an angle with respect to the input beam 105, for instance being arranged at 90 degrees relative to the input beam, such that the optical detector 111 receives photons via output beam 109 that scatter 90 degrees relative to the angle of incidence from the input beam 105. In various implementations, the optical detector 111 can be arranged at any suitable angle with respect to the optical emitter 103 and the input beam 105. Additionally or alternatively, a plurality of optical detectors 111 can be arranged at different spatial positions with respect to the fluid sample 107, thereby collecting photons from a range of output angles.

### E. Example Signal Processing Components

The signal processing components 113 in the optical sensor assembly 101 can be configured to convert the raw signal from the optical detector 111 into a meaningful indication of the analyte concentration. These components 113 can optionally include a variety of analog and/or digital circuitry, depending on the specific requirements and constraints of the application.

One common example of a signal processing component 113 is a transimpedance amplifier (TIA). A TIA is an analog circuit that converts the small current signal generated by the optical detector 111 (e.g., a photodiode) into a proportional voltage signal. The TIA also amplifies the signal to a suitable level for further processing, while minimizing noise and maintaining a high signal-to-noise ratio (SNR). The output of the TIA can be fed into an analog-to-digital converter (ADC) to digitize the signal for subsequent digital processing.

The signal processing components 113 can be configured to perform digital signal processing (DSP) techniques, which may be employed to analyze the digitized signal and extract the relevant information about the analyte concentration. For example, in absorption spectroscopy, the DSP components can calculate the absorbance of the sample based on the ratio of the transmitted light intensity to the incident light intensity, and then use the Beer-Lambert law to determine the exact concentration of the analyte. This can be achieved using a microcontroller or a field-programmable gate array (FPGA) running a suitable algorithm, such as a least-squares fitting routine or a machine learning model trained on calibration data.

In the case of dynamic light scattering, the DSP components can perform autocorrelation analysis on the digitized signal to extract the characteristic time scales of the intensity fluctuations, which are related to the size and motion of the analyte particles. By comparing the measured autocorrelation function to theoretical models or calibration data, it is possible to determine the exact concentration of the analyte in the sample. This can be achieved using specialized hardware, such as a digital correlator, or software running on a microprocessor or FPGA.

In some applications, it may be sufficient to sort readings of analyte concentration in the fluid sample 107 into one of a predefined number of concentration ranges, rather than determining the exact concentration value. This can simplify the signal processing requirements and reduce the cost and complexity of the optical sensor assembly 101. For example, in the case of insulin, it may be desirable to distinguish between U100, U200, and U500 formulations, which have different concentrations of insulin per unit volume.

To achieve concentration range sorting, the signal processing components 113 can include a series of analog comparators or digital threshold detectors. These components compare the amplitude or other characteristics of the detector signal to predefined threshold values corresponding to the different concentration ranges. For example, if the detector signal falls below a certain threshold, it may indicate a U100 insulin formulation, while a signal above another threshold may indicate a U500 formulation. The output of the comparators or threshold detectors can be used to drive indicators (e.g., LEDs) or generate digital codes that identify the concentration range of the sample.

Another approach to concentration range sorting is to use pattern recognition techniques, such as principal component analysis (PCA) or linear discriminant analysis (LDA). These techniques can be applied to the digitized detector signal to extract features that are characteristic of the different concentration ranges, and then classify the sample based on its similarity to the reference patterns. This can be implemented using software running on a microprocessor or FPGA, or using specialized hardware accelerators for machine learning.

In various implementations, data gathered from the optical detector 111 can be compared to reference data to determine resulting analyte concentration levels. Such data may be stored in lookup tables, such as pre-calculated arrays that map given input values (e.g., raw or processed signal data from the optical detector 111) to output values (e.g., analyte concentration or other parameter). In some examples, a mathematical model can be stored that represents the relationship between the incoming signals from the optical detector 111 and the resulting analyte concentration. Such mathematical models can include polynomial regression models, neural network models, classification models, or any other suitable techniques.

### F. Example Actions Responsive to Concentration Determinations

The integration of the optical sensor assembly 101 into the fluid delivery device 100 enables the implementation of various automated actions based on the determined analyte concentration. For instance, based on a determined concentration of analyte (e.g., insulin), the fluid delivery device 100 can perform one or more actions automatically. These actions can help ensure user safety, improve the efficacy of the treatment, and enhance the overall user experience. While the automated action can take any suitable form, three examples are described herein: (1) outputting an alarm to the user, (2) automatically adjusting the dispensing mechanism, and (3) inhibiting medicament dispensation.

Outputting an alarm to the user can serve as a safety feature that is triggered when the optical sensor assembly 101 detects a analyte concentration that differs significantly from the expected or programmed value. For example, if the user has loaded a U200 insulin cartridge into a device programmed for U100 insulin, the higher concentration could lead to an overdose if not detected and corrected. In this case, the signal processing components 113 can compare the measured concentration to the expected value and trigger an alarm if the difference exceeds a predetermined threshold (e.g., 20%).

The alarm can be implemented in various forms, such as an audible buzzer, a visual indicator (e.g., a flashing LED or a warning message on a display), or tactile feedback (e.g., a vibration). The alarm can also be accompanied by a message prompting the user to check the cartridge and reprogram the device if necessary. By alerting the user to potential concentration mismatches, the optical sensor assembly 101 can help prevent medication errors and improve patient safety.

Automatically adjusting the dispensing mechanism is another feature enabled by the optical sensor assembly 101. In some cases, the user may intentionally load a cartridge with a different concentration than the one previously used, in order to adjust the dosage or accommodate changes in their treatment plan. In such situations, the optical sensor assembly 101 can detect the new concentration and automatically adjust the dispensing mechanism to maintain the desired dose. For example, if the user switches from a U100 insulin cartridge to a U200 cartridge, the optical sensor assembly 101 can detect the higher concentration and signal the dispensing mechanism to reduce the volume of fluid dispensed per unit time, in order to maintain the same effective dose of insulin. Conversely, if the user switches to a lower concentration cartridge, the dispensing mechanism can be adjusted to increase the volume of fluid dispensed per unit time. This automatic adjustment can be achieved by modifying the control parameters of the dispensing mechanism, such as the stroke volume of a piston pump or the duty cycle of a peristaltic pump.

Inhibiting medicament dispensation is a third safety feature that can be implemented based on the concentration determination by the optical sensor assembly 101. In some cases, the detected concentration may be so far outside the expected range that it indicates a potential hazard to the user, such as a contaminated or degraded medicament. In such situations, the signal processing components can trigger a complete shutdown of the dispensing mechanism to prevent any further delivery of the suspect medicament.

The inhibition of medicament dispensation can be implemented by disabling the power supply to the dispensing mechanism, closing a safety valve in the fluid path, or engaging a mechanical lock to prevent actuation of the mechanism. The inhibition can be accompanied by an alarm and a message to the user, indicating the reason for the shutdown and advising them to replace the cartridge or seek medical attention if necessary. By preventing the delivery of potentially harmful medicaments, the optical sensor assembly 101 can provide an additional layer of protection for the user.

### G. Calibration Assemblies

In some implementations, it may be useful to provide on-device calibration for the optical sensor assembly 101. Figure 3 illustrates a sensor assembly which includes an optical emitter 103, a fluid sample 107, and an optical detector 111, as described previously. The optical emitter 103 is configured to emit photons as an input beam towards the fluid sample 107, which contains the analyte (e.g., insulin) to be analyzed. The optical detector 111 is configured to receive photons from the fluid sample 107 as an output beam and provide a detector signal to the signal processing components (not shown in Figure 3) for determining the concentration of the analyte in the fluid sample 107.

In addition to the optical sensor assembly 101, Figure 3 also depicts a separate calibration assembly 301. The calibration assembly 301 includes its own optical emitter 303, calibration fluid sample 307, and optical receiver 311. The optical emitter 303 and optical receiver 311 of the calibration assembly 301 may be identical to or different from the optical emitter 103 and optical detector 111 of the main sensor assembly 101, depending on the specific design requirements and the optical technique employed (e.g., UV absorption spectroscopy, dynamic light scattering, etc.).

The calibration fluid sample 307 of the calibration assembly 301 may contain a analyte-free fluid, such as a diluent or solvent used to prepare the medicament solution in the fluid sample 107 of the main sensor assembly 101. By using a analyte-free calibration fluid in the calibration assembly 301, a baseline absorption, scattering, or other relevant optical parameter can be determined for the specific fluid and optical setup used in the fluid delivery device 100.

The optical emitter 303 of the calibration assembly 301 emits photons as an input beam towards the calibration fluid sample 307. The photons interact with the analyte-free calibration fluid in the fluid sample 307, and the resulting output beam is received by the optical receiver 311. The signal processing components 113 (not shown in Figure 3) can then analyze the detector signal from the optical receiver 311 to determine the baseline optical parameter (e.g., absorption, scattering) for the analyte-free calibration fluid 307.

This baseline optical parameter obtained from the calibration assembly 301 can be used to calibrate the main sensor assembly 101. By comparing the detector signal from the optical detector 111 of the main sensor assembly 101 to the baseline signal from the optical receiver 311 of the calibration assembly 301, the signal processing components 113 can more accurately determine the concentration of the analyte in the fluid sample 107, taking into account any background absorption, scattering, or other optical effects contributed by the variability in the calibration fluid itself.

The inclusion of a separate calibration assembly 301 in the sensor assembly 101 may help to improve the accuracy and reliability of the analyte concentration measurement, particularly in cases where the fluid composition may vary, such as variations due to temperature or other environmental factors, or where there may be interfering substances present. By regularly calibrating the main sensor assembly 101 using the analyte-free calibration fluid sample 307 in the calibration assembly 301, any drift or changes in the optical properties of the system can be accounted for, ensuring consistent and precise measurements of the analyte concentration in the fluid sample 107.

### H. Absorption Spectroscopy Techniques

As noted previously, in various examples the sensor assembly 101 utilizes ultraviolet (UV) absorption spectroscopy to determine the concentration of analyte, such as insulin, within the fluid sample 107. UV absorption spectroscopy is based on the principle that different substances absorb UV light at specific wavelengths, and the amount of light absorbed is directly proportional to the concentration of the absorbing substance in the sample. In the case of liquid insulin, there is observed an increase in light transmission through the fluid sample at select wavelengths (e.g., 250-350 nm, or about 295 nm) with increasing insulin concentration. This can be attributed to the phenomenon of bathochromic shift. A bathochromic shift, also known as a red shift, occurs when the transmittance maximum of a substance shifts to longer wavelengths (i.e., towards the red end of the spectrum) due to changes in its molecular environment or structure. In the case of insulin solutions, the presence of insulin molecules and their interactions with the solvent can lead to a bathochromic shift in the absorption spectrum of the sample.

As the concentration of insulin in the fluid sample increases, the number of insulin molecules available to interact with the incident light also increases. These interactions can cause a shift in the absorption spectrum of the sample towards longer wavelengths. Consequently, at specific wavelengths, which may be slightly offset from the absorption peak of insulin, the light transmission through the sample increases with increasing insulin concentration for a particular wavelength. This is because the bathochromic shift causes the absorption or transmittance spectra to shift in wavelength, allowing more light to pass through the sample at particular wavelengths. By measuring the light transmission at these carefully selected wavelengths where there is a large change in absorbance or transmittance, the optical sensor assembly 101 can exploit the bathochromic shift phenomenon to accurately determine the insulin concentration in the fluid sample 107.

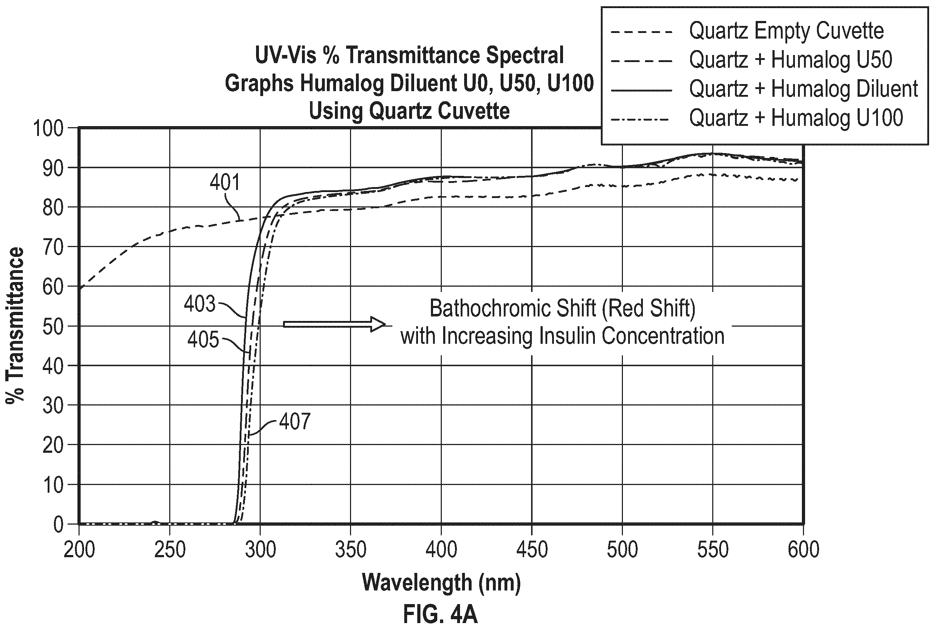

Figure 4A is a graph illustrating the transmittance percent over a range of wavelengths for samples having varying insulin concentrations. Line 401 reflects the percent transmittance for an empty quartz cuvette (e.g., no fluid present within the sample) and line 403 reflects the percent transmittance for a quartz cuvette that includes only diluent (e.g., no insulin present within the fluid sample). Lines 405 and 407 reflect the percent transmittance for quartz cuvettes having a U50 insulin sample and U100 insulin sample, respectively. In each sample that includes fluid within the cuvette (lines 403, 405, and 407), there is a sharp increase in percent transmittance at a given wavelength range. However, this transitional wavelength range varies across the samples, with the transitional wavelength range increasing along with increasing insulin concentration. In other words, the transitional wavelength range is highest for the U100 insulin sample (line 407), then the next highest wavelength range is for the U50 insulin sample (line 405), and finally the lowest transition wavelength range is for the sample with diluent only (line 403). As such, these samples reflect a bathochromic shift, in which the wavelength range at which the transmittance sharply increases is higher for samples with higher insulin concentration.

When the optical emitter 103 emits UV light as the input beam towards the fluid sample 107, the analyte (e.g., insulin) in the sample absorbs a portion of the light. The amount of light absorbed depends on the concentration of the analyte in the fluid sample 107. The remaining light that passes through the sample is received by the optical detector 111 as the output beam.

For absorption spectroscopy, the most suitable orientation between the optical emitter and the optical detector is a straight-line, 180-degree arrangement, also known as a transmission geometry. In this configuration, the optical emitter 103 and the optical detector 111 are positioned directly opposite each other, with the fluid sample 107 placed in between. The optical emitter 103 generates a beam of light that passes through the fluid sample 107, and the optical detector 111 measures the intensity of the transmitted light on the other side. The attenuation of the light intensity as it passes through the sample 107 is directly related to the concentration of the absorbing species (i.e., the analyte) in the fluid.

The transmission geometry is well-suited for absorption spectroscopy because it maximizes the path length of the light through the sample, which enhances the sensitivity of the measurement. Additionally, this orientation minimizes the influence of light scattering, as the detector 111 is positioned in the direct path of the transmitted light from the emitter 103. In this configuration, it can be beneficial to ensure that the fluid sample 107 is sufficiently transparent and free of bubbles or particulates that could scatter or block the light, as this can lead to errors in the concentration measurement.

The signal processing components 113 then compare the intensity of the output beam (measured at the detector 111) to the intensity of the input beam (provided by the emitter 103). The difference in intensities is used to calculate the absorbance of the fluid sample 107 at the specific wavelength emitted by the optical emitter 103. This absorbance can then be used to determine the concentration of analyte (e.g., insulin) based on previously established correlations.

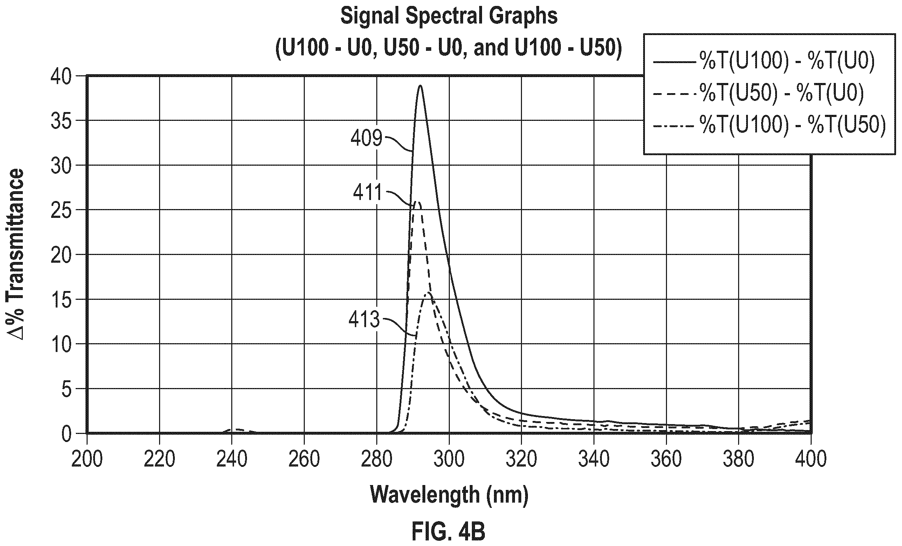

Figure 4B presents a graph depicting the percent difference between the transmittance of light at various wavelengths through different insulin concentrations and a reference sample. The graph compares the precent difference in light transmittance of U100 insulin (100 units of insulin per milliliter of fluid) and U50 insulin (50 units of insulin per milliliter of fluid) as compared with a reference sample U0 (diluent only, no insulin) over a wavelength range from 200 nm to 400 nm. In various examples, the reference sample U0 may be the same as calibration fluid 307 described above with respect to Figure 3.

With continued reference to Figure 4B, line 409 in the graph represents the difference in percent difference in transmittance between the U100 insulin sample and the reference sample (U0) at each wavelength. This line is denoted as "U100 - U0" in the legend. Similarly, line 411 represents the difference in percent transmittance between the U50 insulin sample and the reference sample (U0) at each wavelength, denoted as "U50 - U0" in the legend.

Line 413 in the graph is obtained by subtracting line 411 (U50 - U0) from line 409 (U100 - U0). This line, denoted as "U100 - U50" in the legend, represents the difference in percent transmittance between the U100 insulin sample and the U50 insulin sample at each wavelength.

The graph reveals several key insights into the optical properties of insulin solutions at different concentrations. First, both the line 409 and line 411 show a significant decrease in percent transmittance compared to the reference sample (U0) across the entire wavelength range. This indicates that the presence of insulin in the solution leads to increased transmittance of light at certain wavelengths tested due to the bathochromic shifts of varying insulin concentrations. Because the there is a sharp drop-off in transmittance below 295 nm, even a small shift in the transmittance or absorbance spectra can be used to advantageously detect insulin concentration.

Secondly, line 409 consistently shows a larger increase in the difference in percent transmittance compared to line 411. This suggests that the higher concentration of insulin in the U100 sample results in greater light transmittance than the lower concentration U50 sample.

Additionally, the graph reveals that the greatest difference in percent transmittance between the U100 and U50 insulin samples occurs around the wavelength of 295 nm. This is evident from the peak in line 413 (U100 - U50) at approximately 295 nm. The larger difference in transmittance at this wavelength indicates that light at 295 nm is particularly sensitive to changes in insulin concentration. Depending on the particular analyte (e.g., various formulations of insulin or other medicaments), the particular wavelength of highest sensitivity may vary.

This observation has significant implications for the design of the sensor assembly 101 in the fluid delivery device 100. By selecting an optical emitter 103 that produces light at a wavelength within a range of highest sensitivity (e.g., between about 290 to 300 nm), the sensor assembly 101 can maximize its sensitivity to changes in insulin concentration in the fluid sample 107. This increased sensitivity can lead to more accurate and precise measurements of the insulin concentration, ultimately enabling better control and monitoring of insulin delivery to the patient.

### I. Dynamic Light Scattering Techniques

In various embodiments, the sensor assembly 101 utilizes dynamic light scattering (DLS), also known as photon correlation spectroscopy (PCS), to determine the concentration of analyte, such as insulin, within the fluid sample 107. DLS is a technique that analyzes the fluctuations in the intensity of scattered light over time to determine the size and concentration of particles in a sample.

Figure 5 illustrates an example arrangement of an optical sensor assembly 101 configured to utilize DLS techniques. With reference to Figure 5, when the optical emitter 103, which can be a monochromatic light source in such embodiments, emits light as the input beam 105 towards the fluid sample 107, the light interacts with the analyte particles (e.g., insulin molecules) in the sample 107 and is scattered in various directions. The scattered light is then detected by the optical detector 111 as the output beam 109. The optical detector 111 then outputs a detector signal 501 to the signal processing component(s) 113 for processing. The detector signal can take the form of a time-domain signal of photon intensity over time at the detector 111.

For dynamic light scattering, a suitable orientation between the optical emitter and the optical detector is a 90-degree arrangement, also known as a perpendicular or side-scattering geometry. In this configuration, the optical emitter 103 and the optical detector 111 are positioned at right angles to each other, with the fluid sample 107 placed at the intersection of their optical paths. The optical emitter 103 generates a beam of light that illuminates the fluid sample 107, and the optical detector 111 measures the intensity of the scattered light at a 90-degree angle to the incident beam.

The 90-degree orientation is preferred for dynamic light scattering because it minimizes the influence of the incident light on the optical detector 111, as the direct beam passes through the sample 107 without reaching the detector 111. This enables more sensitive detection of the scattered light, which contains information about the size and motion of the analyte particles in the fluid sample. By analyzing the fluctuations in the scattered light intensity over time, it is possible to determine the concentration of the analyte in the fluid, as the scattering intensity is proportional to the number of particles in the sample.

It is worth noting that other scattering angles, such as forward scattering (angles greater than 90 degrees) or backscattering (angles less than 90 degrees), can also be used for dynamic light scattering, depending on the specific requirements and constraints of the application. However, the 90-degree orientation may be preferred because it provides a good balance between sensitivity and practicality, as it minimizes the influence of the incident light while still allowing for efficient collection of the scattered light.

Due to the Brownian motion of the analyte particles in the fluid sample 107, the intensity of the scattered light fluctuates over time. The rate of these fluctuations depends on the size of the particles: smaller particles move more quickly and cause rapid fluctuations, while larger particles move more slowly and cause slower fluctuations. The signal processing components 113, which can include a digital autocorrelator, can analyze the intensity fluctuations of the scattered light over time. The autocorrelator compares the intensity of the scattered light at different time intervals and generates an autocorrelation function, which describes how the intensity fluctuations are related to the size and concentration of the analyte particles in the fluid sample 107.

By fitting the autocorrelation function to mathematical models, the signal processing components 113 can determine the average size of the analyte particles (e.g., insulin molecules) in the fluid sample 107. Since the size of the analyte particles is known (e.g., the size of an insulin molecule is a constant), the signal processing components 113 can use this information to calculate the concentration of the analyte in the fluid sample 107.

The higher the concentration of the analyte in the fluid sample 107, the more particles there are to scatter light, resulting in a higher average intensity of the scattered light detected by the optical detector 111. Conversely, a lower analyte concentration will result in fewer scattering events and a lower average intensity of the scattered light.

### J. Additional Sensing Techniques

In addition to UV absorption spectroscopy and dynamic light scattering, there are several other sensing techniques that the sensor assembly 101 can use to determine the concentration of insulin within a fluid sample 107. These techniques include dielectric spectroscopy, infrared spectroscopy, fluorescence spectroscopy, Raman spectroscopy, and Fourier-transform infrared (FTIR) spectroscopy.

In addition to the optical sensing techniques described above, the optical sensor assembly 101 can also employ dielectric spectroscopy to determine the insulin concentration in the fluid sample 107. Dielectric spectroscopy measures the dielectric properties of a sample, such as its permittivity and conductivity, as a function of frequency. Insulin, being a polar molecule, exhibits distinct dielectric properties that can be exploited for concentration determination. In this technique, the optical emitter 103 is replaced by an electromagnetic wave generator that emits electromagnetic waves at various frequencies towards the fluid sample 107. The optical detector 111 is replaced by a dielectric sensor that measures the sample's response to these electromagnetic waves. By analyzing the frequency-dependent dielectric properties of the fluid sample 107, the signal processing components 113 can determine the insulin concentration. This is possible because the dielectric properties of the sample, such as its permittivity, are influenced by the presence and concentration of insulin molecules. As the insulin concentration increases, the dielectric properties of the sample change in a predictable manner, allowing for accurate concentration determination using the optical sensor assembly 101 adapted for dielectric spectroscopy.

Infrared (IR) spectroscopy is another technique that can be utilized by the optical sensor assembly 101 to determine the insulin concentration in the fluid sample 107. In this method, the optical emitter 103 is an infrared light source that emits light in the IR region of the electromagnetic spectrum, typically between 700 nm and 1 mm. The optical detector 111 is an IR detector that measures the absorption or transmission of IR light through the fluid sample 107. Insulin, like many other organic molecules, absorbs IR light at specific wavelengths due to the vibrational and rotational motions of its chemical bonds. The absorption pattern, known as the IR spectrum, is unique to insulin and can be used as a fingerprint for identification and quantification. As the concentration of insulin in the fluid sample 107 increases, the intensity of the absorption bands in the IR spectrum also increases proportionally. By measuring the absorption or transmission of IR light at these specific wavelengths and comparing the intensities with reference spectra, the signal processing components 113 can accurately determine the insulin concentration in the fluid sample 107. IR spectroscopy offers high sensitivity and specificity, making it a reliable technique for concentration determination using the optical sensor assembly 101.

Fluorescence spectroscopy is based on the principle that certain molecules, called fluorophores, absorb light at a specific wavelength and emit light at a longer wavelength. Insulin, like many proteins, contains intrinsic fluorophores such as tyrosine and tryptophan residues. When excited with light at the appropriate wavelength (usually in the UV range), these fluorophores emit light that can be detected by the optical detector 111. The intensity of the emitted light is proportional to the concentration of insulin in the fluid sample 107. By comparing the intensity of the emitted light to a calibration curve, the signal processing components 113 can determine the insulin concentration.

Raman spectroscopy is a technique that analyzes the inelastic scattering of monochromatic light by molecules in a sample. When the optical emitter 103 emits monochromatic light (usually in the visible or near-infrared range) as the input beam 105 towards the fluid sample 107, some of the light is scattered by the insulin molecules at a different wavelength than the incident light. This shift in wavelength, called the Raman shift, is specific to the molecular structure of insulin and can be used to identify and quantify the insulin concentration in the fluid sample 107. The intensity of the Raman-scattered light, detected by the optical detector 111, is proportional to the insulin concentration.

FTIR spectroscopy is a technique that measures the absorption of infrared light by molecules in a sample. Insulin, like other proteins, has characteristic absorption bands in the infrared region due to the vibrations of its chemical bonds. When the optical emitter 103 emits infrared light as the input beam 105 towards the fluid sample 107, some of the light is absorbed by the insulin molecules at specific wavelengths. The amount of light absorbed is proportional to the insulin concentration in the fluid sample 107. The optical detector 111 detects the transmitted light as the output beam 109, and the signal processing components 113 analyze the absorption spectrum to determine the insulin concentration.

These techniques, along with UV absorption spectroscopy and dynamic light scattering, provide a range of options for the sensor assembly 101 to determine the concentration of insulin (or other analyte of interest) within the fluid sample 107. The choice of technique may depend on factors such as the desired sensitivity, specificity, and complexity of the sensor assembly 101.

### K. Optical Sources with Filters

Figure 6 illustrates an example arrangement of the optical sensor assembly 101 configured to include an optical filter 601. As noted above, an optical emitter 103 is configured to emit photons as an input beam 105 towards a fluid sample 107. An output beam 109 is generated by interactions between the input beam 105 and the fluid sample 107. An optical detector 111 is configured to receive the output beam 109. In the illustrated embodiment, the optical filter 601 is positioned between the optical emitter 103 and the fluid sample 107 such that the input beam 105 passes through the optical filter 601 before interacting with the fluid sample 107. Additionally or alternatively, an optical filter 601 can be incorporated into the optical emitter 103 or can be configured as a separate component.

The optical filter 601 can be used to narrow a large spectral distribution 603 of the optical emitter 103, resulting in a narrow spectral distribution 605 of an altered input beam 607. The narrow spectral distribution 605 will have a center wavelength (CWL), which may be at a wavelength between approximately 250 nanometers and 350 nanometers, between about 275 to about 325 nanometers, between about 285 to about 315 nanometers, between about 290 to about 310 nanometers, or approximately 295 nanometers. In various implementations, the CWL is selected to correspond with absorption characteristics of particular analytes. The specific center wavelength may be selected based on the absorption spectrum of the target analyte, such as insulin or other biological molecules of interest.

The addition of an optical filter 601 to produce the narrow spectral distribution 605 enables the sensor 101 to more effectively distinguish the concentration of analyte in the fluid sample, ultimately improving the control and monitoring of analyte concentration. In some implementations, the optical filter 601 may include a narrow bandpass filter configured to receive ultraviolet radiation having a first spectral distribution and output filtered ultraviolet radiation having a second spectral distribution. The first spectral distribution may exhibit a relatively broad full-width half maximum (FWHM) range, such as between approximately 20 nanometers and 100 nanometers. The optical filter 601 may be configured to substantially reduce the spectral width of the received radiation, whereby the second spectral distribution exhibits a FWHM range between approximately 1 nanometer and 10 nanometers. In some instances, the optical filter 601 can reduce the spectral width (as measured by FWHM) of the received radiation by about 40%, 50%, 60%, 70%, 80%, 90% or more. Additionally or alternatively, the optical filter 601 may comprise a crystalline wavelength filter (CWL) having optical characteristics selected to achieve the desired spectral narrowing while maintaining sufficient optical transmission at the target center wavelength. The narrow spectral distribution of the filtered radiation may enable improved detection sensitivity when analyzing absorption characteristics of an insulin sample or other analyte of interest.

### III. Example Medicament Delivery Devices Incorporating Optical Sensor Assemblies

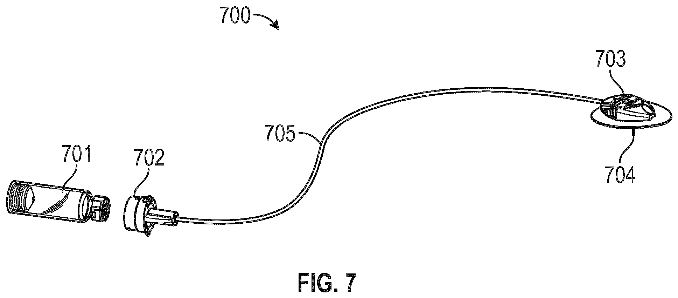

Figures 7, 8, and 9 illustrate various example medicament delivery devices into which the optical sensor assembly 101, as described above, can be integrated. These devices represent different form factors and configurations of medicament delivery devices, each with its own unique features and advantages. However, it should be noted that these are only examples, and the optical sensor assembly 101 can be incorporated into a wide range of other medicament delivery devices, depending on the specific requirements and preferences of the patient and healthcare provider.

The integration of the optical sensor assembly 101 into these devices enables real-time, non-invasive monitoring of the analyte (e.g., medicament) concentration, which can significantly improve the safety, efficacy, and user experience of the medicament delivery process. By continuously measuring the analyte concentration at one or more points within the device's fluid path, the optical sensor assembly 101 can detect any deviations from the expected concentration and alert the user or healthcare provider to potential issues.

Furthermore, the incorporation of the optical sensor assembly 101 into these devices can facilitate the development of closed-loop control systems, where the medicament delivery is automatically adjusted based on the measured analyte concentration and other physiological parameters of the patient. This can lead to more personalized and optimized therapy, ultimately improving the patient's quality of life and treatment outcomes.

### A. Tethered Pump

Figure 7 illustrates an example medicament delivery device in the form of an infusion set 700 typically used with a tethered pump, which can incorporate the sensor assembly 101 described elsewhere herein. Figure 7 illustrates a reservoir 701 which can be placed in fluid communication with an infusion set 700 via an intervening conduit 705. The conduit 705 fluidly connects a cap 702 to the cannula housing 703. In operation, the reservoir 701 is housed within a pump (not shown), which can be worn by a user and actuated to drive fluid from the reservoir 701, through the cap 702 and along the conduit 705 to the cannula housing 703.

The reservoir 701 is responsible for storing and dispensing the medicament, such as insulin, to the patient. The reservoir 701 is typically coupled to a pumping mechanism for controlling the flow of the medicament, and various electronic components for regulating the pump's operation. The sensor assembly 101 can be integrated into the pump, for example, within the reservoir 701, to monitor the concentration of an analyte within the fluid being dispensed.

The cannula housing 703 is a device that is attached to the patient's body, usually via an adhesive patch, and delivers the medicament from the reservoir 701 into the patient's subcutaneous tissue. The cannula housing 703 typically includes a cannula 704 or needle that is inserted into the patient's skin, as well as a fluid path that connects the cannula 704 or needle to the conduit 705. The sensor assembly 101 can also be incorporated into the infusion set 700, anywhere within the fluid path, to measure an analyte concentration just before the fluid is delivered to the patient, such as in the cap 702 or in the cannula housing 703.

The conduit 705 is a flexible tube that carries the medicament from the reservoir 701 through the cap 702 to the cannula housing 703. It is typically made of a biocompatible material that is resistant to kinking and can withstand the pressure generated by the pump. The sensor assembly 101 can be integrated along the length of the conduit 705 at one or more locations to monitor an analyte concentration as the fluid travels from the reservoir 701 to the cannula housing 703.

### B. Patch Pump

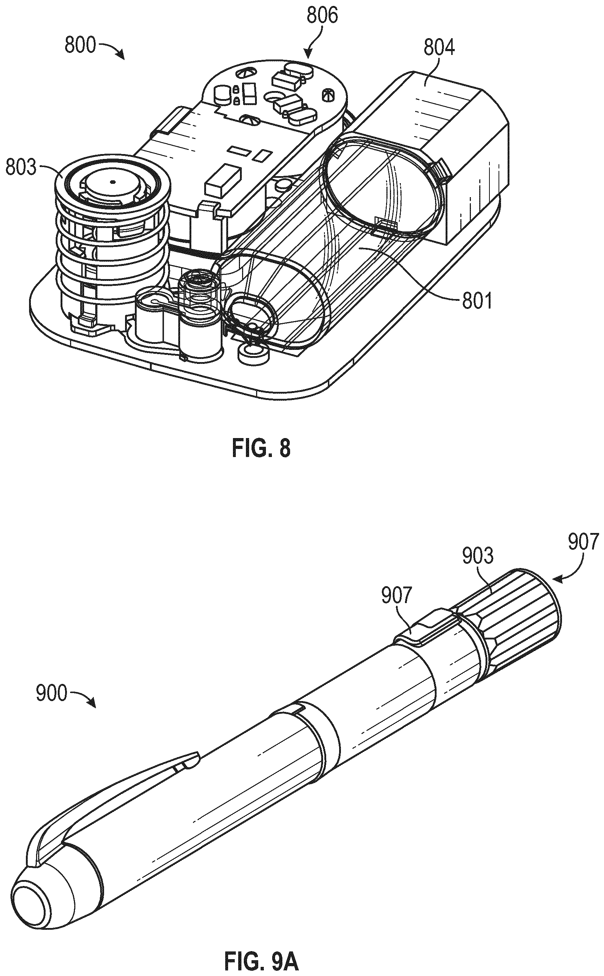

Figure 8 depicts another example medicament delivery device in the form of a patch pump 800, which is designed to be worn directly on the user's skin. For clarity, the patch pump 800 is depicted with an outer shell removed to better illustrate the internal components. The patch pump 800 is a compact, self-contained unit that includes a reservoir 801 for storing the medicament and an inserter mechanism 803 that houses an internal cannula (not shown) for delivering the medicament into the patient's subcutaneous tissue. The patch pump 800 also includes a pump mechanism 804 for driving fluid from the reservoir 801 and additional electronic components 806 for controlling operation of the pump 800. A fluid path 808 extends between the reservoir 801 and the cannula (not shown) within the inserter mechanism 803, and is configured to carry fluid from the reservoir 801 to the internal cannula for subcutaneous delivery to the patient.

The reservoir 801 can hold the medicament supply for an extended period (e.g., up to 7 days or more). The reservoir 801 is usually made of a biocompatible material that can accommodate the medicament volume and withstand the pressure generated by the pump's delivery mechanism. The optical sensor assembly 101 can be integrated into or adjacent the reservoir 801 to monitor an analyte concentration, ensuring that the correct amount of medicament is dispensed throughout the life of the patch pump 800.

The inserter mechanism 803 is responsible for automatically inserting the internal cannula into the patient's skin and establishing a fluid path between the reservoir 801 and the subcutaneous tissue. The inserter mechanism 803 typically includes a spring-loaded needle or blade that punctures the skin and guides the cannula into place. Once the cannula is inserted, the needle or blade is automatically retracted, leaving the soft cannula in the subcutaneous tissue to deliver the medicament. The optical sensor assembly 101 can also be incorporated into the inserter mechanism 803, near the cannula, to measure an analyte concentration just before the fluid enters the patient's body.

In various examples, the optical sensor assembly 101 can be incorporated into or adjacent the fluid path 808 between the reservoir 801 and the internal cannula (not shown) within the inserter mechanism 803. Such an arrangement can beneficially enable determination of an analyte concentration within the fluid when the fluid path 808 is filled or primed, which is often performed before the pump 800 is attached to the patient. In this manner, an alert, notification, disabling of the pump 800, or other suitable action can be performed before the pump 800 is attached to the patient.

The patch pump 800 is designed to be worn continuously for a specified duration, usually 2-3 days, before being replaced with a new one. During this time, the pump's electronic components 806, including a battery, a microcontroller, and a wireless communication module, control the medicament delivery according to the programmed therapy settings. The optical sensor assembly 101 can provide real-time feedback to the pump's control system, enabling it to adjust the delivery rate or alert the user if any issues with the medicament concentration are detected.

### C. Pen Injector

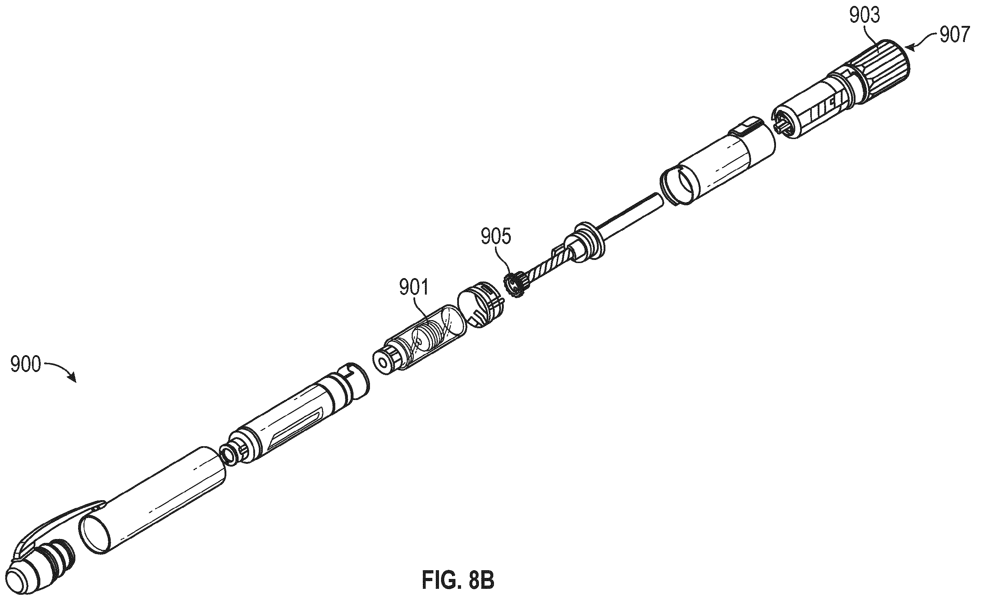

Figures 9A and 9B illustrate yet another example medicament delivery device, a pen injector 900, which is commonly used for self-administered medicament delivery, particularly insulin. The pen injector 900 is a compact, portable device that combines a reservoir 901 for storing the medicament and a built-in mechanism for accurately dispensing the medicament through a needle (not shown).

Figure 9A provides a perspective view of the fully assembled pen injector 900 and Figure 9B illustrates an exploded view of the pen injector, revealing its internal components and potential integration sites for the optical sensor assembly 101. The pen injector 900 typically features a dosage selector 903 at one end, which enables the user to set the desired amount of medicament to be delivered. The dosage selector 903 is usually coupled to a plunger or a screw mechanism 905 that advances the medicament from the reservoir 901 through the needle when the user presses the injection button 907.

The reservoir 901 is typically designed to hold the medicament supply for multiple doses. The reservoir 901 can be a cylindrical cartridge made of glass or a transparent, biocompatible plastic material. The optical sensor assembly 101 can be integrated into or adjacent the reservoir 901 to monitor the medicament concentration, ensuring that the correct amount of medicament is dispensed with each dose.

The integration of the optical sensor assembly 101 into the reservoir 901 of the pen injector 900 offers several advantages. By continuously monitoring the medicament concentration, the optical sensor assembly 101 can detect any deviations in medicament concentration from expected values. This information can be used to alert the user or healthcare provider, preventing the administration of compromised medicament and ensuring the safety and efficacy of the treatment.

### IV. Conclusion

Although the devices and methods are described in the context of medicament infusion devices such as insulin pumps and pen injectors, it should be appreciated that the techniques are equally applicable to a variety of fluid delivery devices, whether other medical devices and/or non-medical devices that deliver fluid. For example, the present technology may also be applicable to devices used to deliver other medicaments such as, for example, drugs to mask pain, chemotherapy and other cancer related drugs, antibiotics, hormones, GLP-1, glucagon, various other drugs that include large molecules and proteins that may require a high level of delivery accuracy. The present technology may also be applicable to devices used to deliver non-medicament fluids.

The descriptions of embodiments of the technology are not intended to be exhaustive or to limit the technology to the precise form disclosed above. Where the context permits, singular or plural terms may also include the plural or singular term, respectively. Although specific embodiments of, and examples for, the technology are described above for illustrative purposes, various equivalent modifications are possible within the scope of the technology, as those skilled in the relevant art will recognize. For example, while steps are presented in a given order, alternative embodiments may perform steps in a different order. The various embodiments described herein may also be combined to provide further embodiments.

As used herein, the terms "generally," "substantially," "about," and similar terms are used as terms of approximation and not as terms of degree, and are intended to account for the inherent variations in measured or calculated values that would be recognized by those of ordinary skill in the art.

Moreover, within the detailed description, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded. It will also be appreciated that specific embodiments have been described herein for purposes of illustration, but that various modifications may be made without deviating from the technology. Further, while advantages associated with certain embodiments of the technology have been described in the context of those embodiments, other embodiments may also exhibit such advantages, and not all embodiments need necessarily exhibit such advantages to fall within the scope of the technology. Accordingly, the disclosure and associated technology can encompass other embodiments not expressly shown or described herein.

The following examples are illustrative of the techniques described herein.

Example 1:A medicament delivery device comprising:
a fluid path comprising a reservoir in fluid communication with an outlet port, wherein the device is actuatable to drive fluid through the fluid path, from the reservoir and out of the outlet port;
a sensor assembly configured to determine concentration of an analyte in the fluid, the sensor assembly comprising:
   an optical emitter configured to emit photons towards a fluid sample within the fluid path;
   an optical detector configured to receive photons passed through and/or reflected from the fluid sample and provide a detector signal; and
   one or more signal processing components configured to:
      receive the detector signal; and
      based at least in part on the detector signal, provide an indication of the analyte concentration in the fluid sample.

Example 2: The medicament delivery device of example 1, wherein the analyte comprises insulin.

Example 3: The medicament delivery device of example 1, wherein the optical emitter is configured to emit photons within a wavelength range between about 250-350 nm.

Example 4: The medicament delivery device of example 1, wherein the device further comprises an optical filter.

Example 5: The medicament delivery device of example 1, wherein the detector is configured to detect photons passing through the fluid sample, and wherein the signal processing components are configured to provide the indication of analyte concentration based on absorption of the photons by the fluid sample.

Example 6: The medicament delivery device of example 5, wherein a greater absorption of the photons by the fluid sample indicates a lower concentration of the analyte in the fluid sample.

Example 7: The medicament delivery device of example 1, wherein the detector is configured to detect scattered photons reflected from medicament within the fluid sample over time to produce a time-domain signal, and wherein the signal processing components are configured to provide the indication of analyte concentration based on the time-domain signal.

Example 8: The medicament delivery device of example 7, wherein the one or more signal processing components comprises a digital autocorrelator that correlates intensity fluctuations in detected photons over time.

Example 9: The medicament delivery device of example 1, further comprising a reference sample that is separate from the fluid path for calibrating the sensor assembly, wherein the reference sample contains fluid with no medicament therein.

Example 10: The medicament delivery device of example 9, wherein the reference sample is housed within a receptacle made of the same material as a portion of the fluid path containing the fluid sample.

Example 11: The medicament delivery device of example 1, wherein the optical emitter is configured to emit photons within a predefined wavelength range, and wherein portion of the fluid path containing the fluid sample comprises a material substantially translucent to photons within the predefined wavelength range.

Example 12: The medicament delivery device of example 1, wherein the signal processing components are further configured to, based at least in part on the indication of the analyte concentration in the fluid sample:
cause an alarm to be output to a user;
cause a dispensation mechanism to be adjusted, wherein the adjustment varies an amount or rate of fluid dispensed via the medicament delivery device; and/or
inhibit dispensation of fluid via the medicament delivery device.

Example 13: The medicament delivery device of example 1, wherein the delivery device comprises a wearable pump.

Example 14: The medicament delivery device of example 1, wherein the delivery device comprises a pen injector.

Example 15: A method comprising:
disposing fluid comprising medicament within a fluid path of a medicament delivery device;
directing photons towards a fluid sample within the fluid path;
detecting photons transmitted through and/or scattered from the fluid sample; and
based on the detected photons, providing an indication of an analyte concentration in the fluid sample.

Example 16: The method of example 15, wherein the analyte comprises insulin.

Example 17: The method of example 15, wherein the directing photons towards the fluid sample comprises directing photons within a wavelength range of between about 250-350 nm.

Example 18: The method of example 15, wherein the directing photons towards the fluid samples comprises directing photons through an optical filter.

Example 19: The method of example 15, wherein providing the indication of analyte concentration in the fluid sample based on the detected photons comprises determining the analyte concentration based on absorption of the photons by the fluid sample.

Example 20: The method of example 15, wherein providing the indication of analyte concentration in the fluid sample based on the detected photons comprises determining the analyte concentration based on a time-domain signal of the detected photons.

Example 21: The method of example 15, further comprising, based at least in part on the indication of the analyte concentration in the fluid sample:
causing an alarm to be output to a user;
causing a dispensation mechanism to be adjusted, wherein the adjustment varies an amount or rate of fluid dispensed via the medicament delivery device; and/or
inhibit dispensation of fluid via the medicament delivery device.

Example 22: A device comprising:
a means for containing a fluid comprising a medicament;
a means for dispensing the fluid from the fluid-containing means; and
means for sensing an analyte concentration of the fluid, the sensing means comprising:
   means for emitting photons towards a fluid sample within the device;
   means for detecting photons from the fluid sample within the device; and
   means for processing a signal from the detecting means and providing an indication of the analyte concentration.

Example 23: The device of example 22, wherein the means for emitting photons comprises a light source.

Example 24: The device of example 22, wherein the means for detecting photons comprises an optical detector.

Example 25: The device of example 22, wherein the means for processing signals comprises signal processing circuitry.

## Claims

1. A medicament delivery device comprising:
a fluid path comprising a reservoir in fluid communication with an outlet port, wherein the device is actuatable to drive fluid through the fluid path, from the reservoir and out of the outlet port;
a sensor assembly configured to determine concentration of an analyte in the fluid, the sensor assembly comprising:
an optical emitter configured to emit photons towards a fluid sample within the fluid path;
an optical detector configured to receive photons passed through and/or reflected from the fluid sample and provide a detector signal; and
one or more signal processing components configured to:
receive the detector signal; and
based at least in part on the detector signal, provide an indication of the analyte concentration in the fluid sample.

2. The medicament delivery device of claim 1, wherein the analyte comprises insulin.

3. The medicament delivery device of any one of the preceding claims, wherein the optical emitter is configured to emit photons within a wavelength range between about 250-350 nm.

4. The medicament delivery device of any one of the preceding claims, wherein the detector is configured to detect photons passing through the fluid sample, and wherein the signal processing components are configured to provide the indication of analyte concentration based on absorption of the photons by the fluid sample.

5. The medicament delivery device of claim 4, wherein a greater absorption of the photons by the fluid sample indicates a lower concentration of the analyte in the fluid sample.

6. The medicament delivery device of any one of claims 1-3, wherein the detector is configured to detect scattered photons reflected from medicament within the fluid sample over time to produce a time-domain signal, and wherein the signal processing components are configured to provide the indication of analyte concentration based on the time-domain signal.

7. The medicament delivery device of claim 6, wherein the one or more signal processing components comprises a digital autocorrelator that correlates intensity fluctuations in detected photons over time.

8. The medicament delivery device of any one of the preceding claims, further comprising a reference sample that is separate from the fluid path for calibrating the sensor assembly, wherein the reference sample contains fluid with no medicament therein.

9. The medicament delivery device of claim 8, wherein the reference sample is housed within a receptacle made of the same material as a portion of the fluid path containing the fluid sample.

10. The medicament delivery device of any one of the preceding claims, wherein the optical emitter is configured to emit photons within a predefined wavelength range, and wherein portion of the fluid path containing the fluid sample comprises a material substantially translucent to photons within the predefined wavelength range.

11. The medicament delivery device of claim 10, wherein the material comprises at least one of: quartz or topaz.

12. The medicament delivery device of any one of the preceding claims, wherein the signal processing components are further configured to:
based at least in part on the indication of the analyte concentration in the fluid sample, cause an alarm to be output to a user.

13. The medicament delivery device of any one of the preceding claims, wherein the signal processing components are further configured to:
based at least in part on the indication of the analyte concentration in the fluid sample, cause a dispensation mechanism to be adjusted, wherein the adjustment varies an amount or rate of fluid dispensed via the medicament delivery device.

14. The medicament delivery device of any one of the preceding claims, wherein the signal processing components are further configured to:
based at least in part on the indication of the analyte concentration in the fluid sample, inhibiting dispensation of fluid via the medicament delivery device.

15. The medicament delivery device of any one of the preceding claims, wherein the delivery device comprises a wearable pump.
